# EUROPEAN PATENT APPLICATION

(11) **EP 4 173 560 A1**
(43) Date of publication of application: **03.05.2023**
(21) Application number: 21829216.7
(22) Date of filing: 09.04.2021
(51) Int. Cl.: A61B 5/053, A61B 5/0295, A61B 5/00

(54) **METHOD AND APPARATUS FOR CALCULATING HEMODYNAMIC VARIABLE BY USING ELECTRICAL IMPEDANCE TOMOGRAPHY**

(30) Priority: 25.06.2020 KR 20200077738
(71) Applicant: Bilab Co., Ltd., Seongnam-si, Gyeonggi-do 13486 (KR)
(72) Inventor: JANG, Geuk Young, Seoul 02458 (KR); WI, Hun, Suwon-si Gyeonggi-do 16712 (KR); CHANG, Jun, Seoul 08014 (KR)
(74) Representative: Michalski Hüttermann & Partner Patentanwälte mbB
(86) International application number: PCT/KR2021/004458
(87) International publication number: WO 2021/261721

(57) **Abstract**

Provided is a method for calculating a hemodynamic variable for a subject on the basis of electrical impedance tomography (EIT). The present method may comprise the steps of: obtaining EIT images at discrete time points in a cardiac cycle of a subject by targeting a chest portion of the subject; identifying, in the EIT images, values of a first pixel in a region corresponding to the heart and values of a second pixel in a region corresponding to a lung, wherein the values of the first pixel are associated with the discrete time points, respectively, and the values of the second pixel are associated with the discrete time points, respectively; and calculating at least one hemodynamic variable on the basis of a first time point associated with a minimum value among the identified values of the first pixel and a second time point associated with a maximum value among the identified values of the second pixel.

## Description

### [Technical Field]

The present disclosure relates to the field of biomedical engineering, in more detail, a technology of non-invasively monitoring a hemodynamic variable of an examinee.

### [Background Art]

A pulmonary artery, which is an artery connecting the right ventricle of a heart and lungs to each other, is difficult to approach. Accordingly, an invasive method of inserting a catheter into a pulmonary artery through a central vein and the right atrium and the right ventricle of a heart is used to measure pulmonary artery pressure (PAP). In this case, Swan-Ganz catheter is the most generally used, a skillful clinician is required for a surgical procedure of inserting Swan-Ganz catheter through the right atrium and the right ventricle, and it has been known that even though a catheter is successfully inserted, a heart is burdened, which causes various side effects.

It is possible to solve this problem when measuring PAP using an invasive method, so many studies have been conducted. In particular, various methods using echocardiography have been proposed. However, according to echocardiography, since a user manually puts an ultrasound probe on the skin and measures PAP, the result depends on the skillfulness of users and fluctuation when the same user repeatedly measures PAP is also large. Further, continuous measurement is difficult, so these methods are very inconvenient when they are used for monitoring.

In order to solve these problems, many studies about hemodynamic monitoring method including PAP that uses electrical impedance tomography (EIT) have been conducted. When EIT data are measured from the chest of a human body, a signal about respiration by the lungs and a signal about the blood flow in the heart are mixed in the data. Since the intensity of a respiration signal is generally over 10 times the intensity of a blood flow signal, it is very difficult to extract a blood flow signal. Further, since blood flow quickly changes in comparison to respiration, it is required to increase the EIT data collection speed.

One of the difficult subjects of hemodynamic monitoring is to invasively measure PAP. Some techniques of invasively measuring PAP have been known in the art, but these techniques are all evaluated as causing errors in PAP measurement. According to knowledge known in the field of hemodynamics, PAP depends on a right ventricular ejection time (RVET) that is time that the right ventricle of a heart takes to send blood to a pulmonary artery through contraction, a pulmonary perfusion time for which blood is supplied to the lungs, one-time stroke volume (SV) of a ventricle, a variation of a one-time stroke volume (DSV), etc., but existing techniques do not consider this fact.

### [Disclosure]

### [Technical Problem]

An objective of the preset disclosure is to provide an improved method and apparatus for non-invasively monitoring a hemodynamic variable by using electrical impedance tomography.

The objectives of the present disclosure are not limited to the objects described above and other objectives will be clearly understood by those skilled in the art from the following description.

### [Technical Solution]

An aspect provides a method of calculating a hemodynamic variable of an examinee on the basis of Electrical Impedance Tomography (EIT). The method includes: obtaining EIT images of a chest of an examinee at discrete points in time in a cardiac cycle of the examinee; recognizing values of a first pixel (position) in a region corresponding to a heart in the EIT images and values of a second pixel (position) in a region corresponding to lungs - the values of the first pixel are related to the discrete points in time, respectively, and the values of the second pixel are related to the discrete points in time, respectively; and calculating at least one hemodynamic value on the basis of a first point in time related to a minimum value of the recognized values of the first pixel and a second point in time related to a maximum value of the recognized values of the second pixel.

In an embodiment, the obtaining of EIT images of a chest of an examinee at discrete points in time in a cardiac cycle of the examinee may include: attaching a plurality of electrodes to the chest of the examinee and obtaining impedance data for the chest of the examinee; and restoring the EIT images from the impedance data.

In an embodiment, the recognizing of values of a first pixel in a region corresponding to a heart in the EIT images and values of a second pixel in a region corresponding to lungs - the values of the first pixel are related to the discrete points in time, respectively, and the values of the second pixel are related to the discrete points in time, respectively -, may include designating the region corresponding to the heart in the EIT images as a first region of interest and selecting the first pixel in the first region of interest, and designating the region corresponding to the lungs in the EIT images as a second region of interest and selecting the second pixel in the second region of interest.

In an embodiment, the first point in time may be ventricular ejection time and the second point in time may be pulmonary perfusion time.

In an embodiment, the calculating of at least one hemodynamic value on the basis of a first point in time related to a minimum value of the recognized values of the first pixel and a second point in time related to a maximum value of the recognized values of the second pixel may include calculating pulmonary artery pressure (PAP) on the basis of the first point in time and the second point in time.

In an embodiment, the calculating of pulmonary artery pressure (PAP) on the basis of the first point in time and the second point in time may include calculating the pulmonary artery pressure on the basis of the first point in time, the second point in time, the minimum value, and the maximum value.

In an embodiment, the values of the second pixel may show two or more peak patterns, and the calculating of pulmonary artery pressure (PAP) on the basis of the first point in time and the second point in time may include calculating the pulmonary artery pressure on the basis of the first point in time, the second point in time, the minimum value, the maximum value, peak values of the second pixel at the other peak patterns excluding a peak pattern related to the maximum value of the two or more peak patterns, and points in time related to the peak values.

In an embodiment, the values of the first pixel may show two or more peak patterns, and the calculating of pulmonary artery pressure (PAP) on the basis of the first point in time and the second point in time may include calculating the pulmonary artery pressure on the basis of the first point in time, the second point in time, the minimum value, the maximum value, peak values of the first pixel at the other peak patterns excluding a peak pattern related to the minimum value of the two or more peak patterns, and points in time related to the peak values.

In an embodiment, the calculating of pulmonary artery pressure (PAP) on the basis of the first point in time and the second point in time may include calculating the pulmonary artery pressure on the basis of the first point in time, the second point in time, electrocardiography (ECG) of the examinee, blood pressure of the examinee, photoplethysmography (PPG) of the examinee, and seismocardiography (SCG) of the examinee.

In an embodiment, the calculating of at least one hemodynamic value on the basis of a first point in time related to a minimum value of the recognized values of the first pixel and a second point in time related to a maximum value of the recognized values of the second pixel may further include calculating pulmonary vascular resistance (PVR) using the calculated pulmonary artery pressure.

In an embodiment, the calculating of at least one hemodynamic value on the basis of a first point in time related to a minimum value of the recognized values of the first pixel and a second point in time related to a maximum value of the recognized values of the second pixel may include calculating myocardial contractility on the basis of the minimum value and the first point in time.

In another aspect, a method of calculating a hemodynamic variable of an examinee on the basis of EIT is provided. The method includes: obtaining EIT images of a chest of an examinee at discrete points in time in a plurality of cardiac cycles of the examinee; recognizing a pixel value corresponding to a first point in time of pixel values in a region corresponding to a heart in EIT images obtained at discrete points in times in a first cardiac cycle of a plurality of cardiac cycles, and a pixel value corresponding a second point in time of pixel values in a region corresponding to the heart of EIT images obtained at discrete points in time in a second cardiac cycle of the plurality of cardiac cycles - the first point in time is advanced further than the second point in time in terms of time by time corresponding to the cardiac cycle; and calculating at least one hemodynamic value on the basis of the pixel value corresponding to the first point in time and the pixel value corresponding to the second point in time.

In an embodiment, the first point in time and the second point in time may be end diastole points in time, and the calculating of at least one hemodynamic value on the basis of the pixel value corresponding to the first point in time and the pixel value corresponding to the second point in time may include calculating a variation of end-diastolic ventricular volume (EDW) on the basis of the pixel value corresponding to the first point in time and the pixel value corresponding to the second point in time.

In an embodiment, the calculating of at least one hemodynamic value on the basis of the pixel value corresponding to the first point in time and the pixel value corresponding to the second point in time may further include calculating myocardial contractility using the calculated variation of end-diastolic ventricular volume and a variation of a one-time stroke volume (SV) between cardiac cycles.

In an embodiment, the first point in time and the second point in time may be end systole points in time, and the calculating of at least one hemodynamic value on the basis of the pixel value corresponding to the first point in time and the pixel value corresponding to the second point in time may include calculating a variation of end-systolic ventricular volume (ESW) on the basis of the pixel value corresponding to the first point in time and the pixel value corresponding to the second point in time.

In an embodiment, the calculating of at least one hemodynamic value on the basis of the pixel value corresponding to the first point in time and the pixel value corresponding to the second point in time may further include calculating ejection fraction (EF) using the calculated variation of end-systolic ventricular volume and a variation of a one-time stroke volume between cardiac cycles.

In another aspect, an apparatus for calculating a hemodynamic variable of an examinee on the basis of EIT is provided. The apparatus includes: a storage configured to store EIT images - the EIT images are images of a chest of an examinee obtained at discrete points in time in a cardiac cycle of the examinee; and a controller configured to recognize values of a first pixel in a region corresponding to a heart in the EIT images and values of a second pixel in a region corresponding to lungs - the values of the first pixel are related to the discrete points in time, respectively, and the values of the second pixel are related to the discrete points in time, respectively -, and configured to calculate at least one hemodynamic value on the basis of a first point in time related to a minimum value of the recognized values of the first pixel and a second point in time related to a maximum value of the recognized values of the second pixel.

In an embodiment, the first point in time may be ventricular ejection time and the second point in time may be pulmonary perfusion time.

In an embodiment, the controller may be further configured to calculate pulmonary artery pressure on the basis of the first point in time and the second point in time.

In an embodiment, the controller may be further configured to the pulmonary artery pressure on the basis of the first point in time, the second point in time, the minimum value, and the maximum value.

In an embodiment, the controller may be further configured to calculate myocardial contractility on the basis of the minimum value and the first point in time.

In another aspect, an apparatus for calculating a hemodynamic variable of an examinee on the basis of EIT is provided. The apparatus includes: a storage configured to store EIT images - the EIT images are images of a chest of an examinee obtained at discrete points in time in a plurality of cardiac cycles of the examinee; and a controller configured to recognize a pixel value corresponding to a first point in time of pixel values in a region corresponding to a heart in EIT images obtained at discrete points in times in a first cardiac cycle of a plurality of cardiac cycles, and a pixel value corresponding a second point in time of pixel values in a region corresponding to the heart of EIT images obtained at discrete points in time in a second cardiac cycle of the plurality of cardiac cycles - the first point in time is advanced further than the second point in time in terms of time by time corresponding to the cardiac cycle -, and configured to calculate at least one hemodynamic value on the basis of the pixel value corresponding to the first point in time and the pixel value corresponding to the second point in time.

In an embodiment, the first point in time and the second point in time may be end diastole points in time, and the controller may be further configured to calculate a variation of end-diastolic ventricular volume on the basis of the pixel value corresponding to the first point in time and the pixel value corresponding to the second point in time.

In an embodiment, the first point in time and the second point in time may be end diastole points in time, and the controller may be further configured to calculate a variation of end-systolic ventricular volume on the basis of the pixel value corresponding to the first point in time and the pixel value corresponding to the second point in time.

### [Advantageous Effects]

According to embodiments of the present disclosure, there is an effect that it is possible to non-invasively monitor hemodynamic variables by using electrical impedance tomography.

### [Description of Drawings]

FIG. 1 is a block diagram of an embodiment of an apparatus for calculating a hemodynamic variable of an examinee on the basis of electrical impedance tomography (EIT) .
FIG. 2(a) is a diagram showing an embodiment of an EIT image obtained from the chest of an examinee.
FIG. 2(b) is a diagram showing an embodiment of an EIT image obtained from the chest of an examinee.
FIG. 3 is a diagram exemplifying a pattern in which values of a first pixel and values of a second pixel change in accordance with discrete points in time for one cardiac cycle of an examinee.
FIG. 4 is a diagram showing changes over time of a ventricular ejection time TD_{H}, a pulmonary perfusion time TD_{L}, a mean transit time MTT, and pulmonary artery pressure PAP during a plurality of cardiac cycles.
FIG. 5 is a diagram exemplifying a pattern in which values of a first pixel and values of a second pixel change in accordance with discrete points in time for two cardiac cycles of an examinee.
FIG. 6 is a flowchart showing a first embodiment of a method of calculating a hemodynamic variable of an examinee on the basis of ETI.
FIG. 7 is a flowchart showing a second embodiment of a method of calculating a hemodynamic variable of an examinee on the basis of ETI.

### [Best Mode for Disclosure]

The advantages and features of the present disclosure, and methods of achieving them will be clear by referring to the exemplary embodiments that will be describe hereafter in detail with reference to the accompanying drawings. However, the present disclosure is not limited to the exemplary embodiments described hereafter and may be implemented in various ways, and the exemplary embodiments are provided to complete the description of the present disclosure and let those skilled in the art completely know the scope of the present disclosure and the present disclosure is defined by claims.

Terms used in the present disclosure are used only in order to describe specific exemplary embodiments rather than limiting the present disclosure. For example, a component expressed as a singular should be understood as a concept including a plurality of components as long as it clearly means only a singular. Further, in the specification of the present disclosure, terms "include" or "have" only show that specific feature, number, step, operation, component, part, or a combination thereof described in the specification exist without excluding the possibility of existence or addition of one or more other features, numbers, steps, operations, components, parts, or combinations thereof. In embodiments described herein, a "module" or a "unit" may mean a functional part that performs at least one function or operation.

Unless otherwise defined, all terms including technical and scientific terms used herein have the same meaning as commonly understood by those skilled in the art to which the present disclosure belongs. It will be further understood that terms defined in dictionaries that are commonly used should be interpreted as having meanings that are consistent with their meanings in the context of the relevant art and will not be interpreted in an idealized or overly formal sense unless expressly so defined herein.

Hereinafter, embodiments of the present disclosure are described in detail with reference to the accompanying drawings. However, in the following description, it is to be noted that, when the functions of conventional elements and the detailed description of elements may make the gist of the present disclosure unclear, a detailed description of those elements will be omitted.

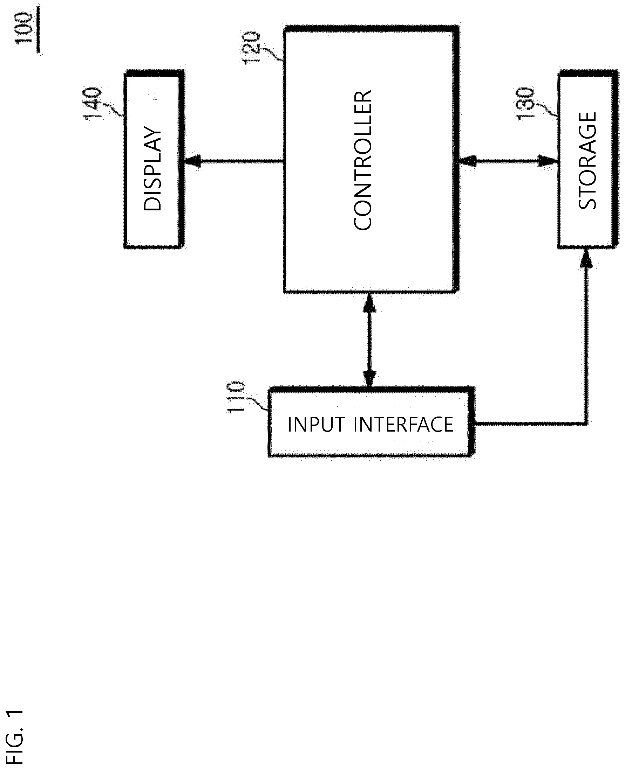

FIG. 1 is a block diagram of an embodiment of an apparatus for calculating a hemodynamic variable of an examinee on the basis of electrical impedance tomography (EIT) .

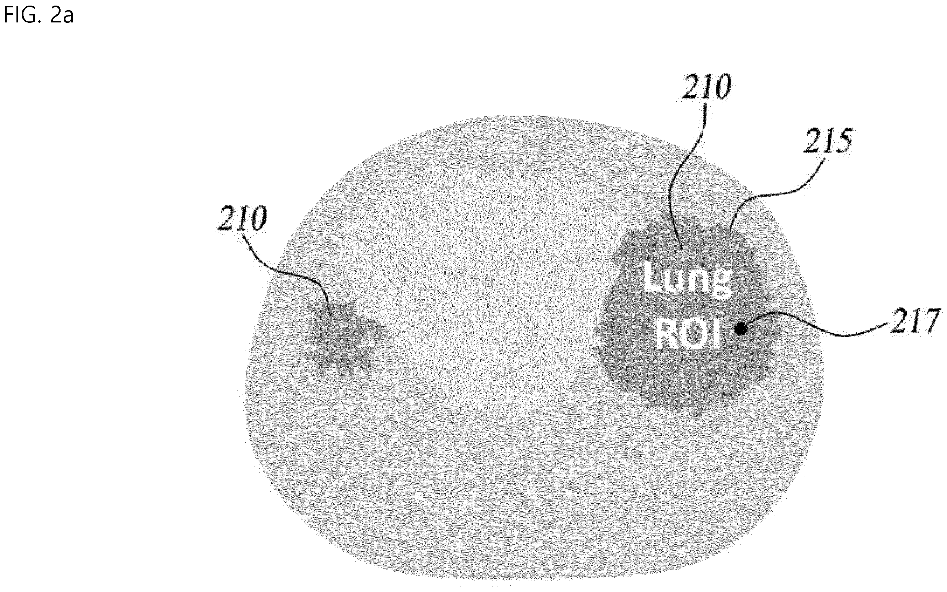

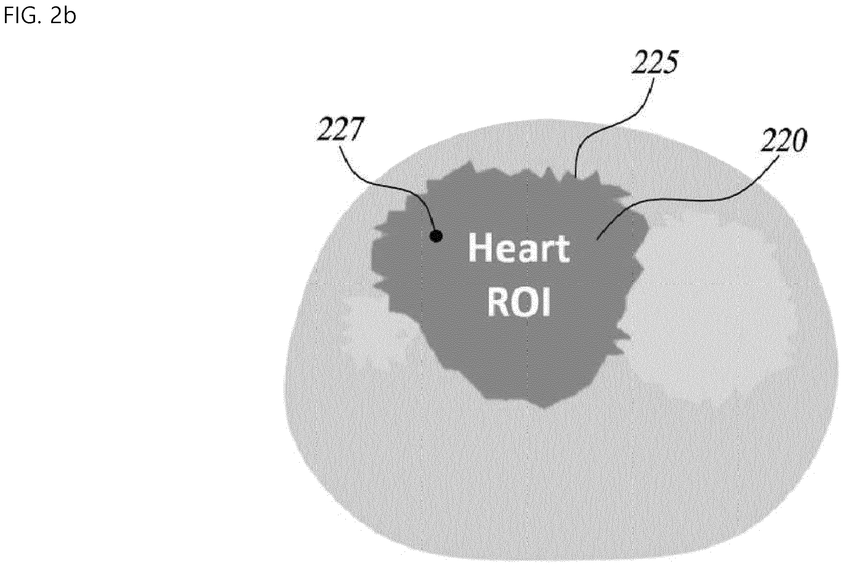

As shown in FIG. 1, the apparatus 100 may include an input interface 110, a controller 120, a storage 130, and a display 140. The storage 130 can store EIT images obtained for the chest of an examinee. In an embodiment, stored EIT images may be images of the chest of an examinee obtained at discrete points in time in one cardiac cycle of the examinee. In an embodiment, stored EIT images may be images of the chest of an examinee at discrete points in time during a plurality of cardiac cycles of the examinee. Stored EIT images may be obtained by attaching a plurality of electrodes to the chest of an examinee, obtaining impedance data for the chest of the examinee, and restoring the obtained impedance data. Referring to FIGS. 2(a) and 2(b) showing embodiments of EIT images obtained for the chest of an examinee, stored EIT images may include an image region 210 including lungs and an image region showing a heart. A cardiac cycle of an examinee may be determined by measuring the cycle of R waves of an electrocardiography (ECG) cycle obtained from the examinee. The cycle of discrete points in time at which EIT images are obtained may be determined by system resolution. For example, when there is a system that obtained 100 sheets of EIT images per second, the system resolution is 10ms and one sheet of EIT image is obtained at every 10ms, so the cycle of the discrete points in time at which the EIT images are obtained may be 10ms.

The storage 130 may be used to store image data of an intermediate result obtained through image processing in accordance with various embodiments of the present disclosure, result image data obtained through image processing in accordance with various embodiments of the present disclosure, variable values for image processing and/or operating according to various embodiment of the present disclosure, pixel data selected from EIT images in accordance with various embodiments of the present disclosure, and operating result values obtained by operating in accordance with various embodiment of the present disclosure. In various embodiments, the storage 130 may store EIT images in the format of Digital Imaging and Communications in Medicine (DICOM) or common image file formats (BMP, JPEG, TIFF, etc.). The storage 130 may further store software/firmware, etc. for implementing the controller 120. The storage may be any one storage medium of a flash memory type, a hard disk type, a MultiMedia Card (MMC) type, a card type memory (e.g., Secure Digital (SD) card or an xTream Digital (XD) card), a Random Access Memory (RAM), a Static Random Access Memory (SRAM), a Read-Only Memory (ROM), an Electrically Erasable Programmable Read-Only Memory (EEPROM), a Programmable Read-Only Memory (PROM), a magnetic memory, a magnetic disc, and an optical disc, but those skilled art would know that the storage 130 is not limited thereto.

The input interface 110 may be a hardware or software module for inputting user instructions to perform image processing and/or operating according to various embodiments of the present disclosure. The input interface 110 may be usefully used to input various necessary instructions to the controller 120, input various image data such as EIT image data obtained by an EIT imaging device to the controller 130, or and perform various types of image processing by designating some or all of displayed images. The input interface 110 may be usefully used to designate a specific region in an EIT image as a region of interest (ROI) and select any point in the designated region of interest. In an embodiment, the input interface 110 may include a keyboard, a keypad, a touchpad, a mouse, etc. of a computer, but the kind of the input interface is not limited thereto. For example, the input interface 110 may include a graphic user interface that can control the input devices described above. The display 140, which is for visually displaying various images and/or various data according to various embodiments of the present disclosure, may include various display devices such as an LCD display, an LED display, an AMOLED display, and a CRT display.

The controller 120 may configured to discriminate the values of a first pixel (position) in a region corresponding to a heart and the values of a second pixel (position) in regions corresponding to lungs in EIT images obtained at a plurality of discrete points in time, respectively. In an embodiment, the controller 120 may be configured to designate a region 220 showing a heart as a first region of interest 225 in EIT images and to select a first pixel 227 in the first region of interest 225. In an embodiment, the controller 120 may be configured to designate regions 210 showing lungs as a second region of interest 215 in EIT images and to select a second pixel 217 in the second region of interest 215. In an embodiment, the controller 120 can designate the first region of interest 225 and the second region of interest 215 using algorithms such as an edge detection algorithm and an image segmentation algorithm, but it should be noted that the method of designating first region of interest 225 and the second region of interest 215 is not limited thereto. For example, a user may manually designate the first region of interest 225 and the second region of interest 215 using the input interface 110. The values of a first pixel and the values of a second pixel are all associated with the discrete points in time at which EIT images were obtained. By this interrelation, it is possible to know the points in time at which the values of the first pixel (position) were obtained such as a first value of the first pixel (position) 227 is a value of the first pixel 227 selected from an EIT image obtained at 15ms and a second value of the first pixel 227 is a value of the first pixel 227 selected from an EIT image obtained at 25ms. Similarly, it is possible to know the points in time at which the values of the second pixel (position) 217 were obtained from the interrelation.

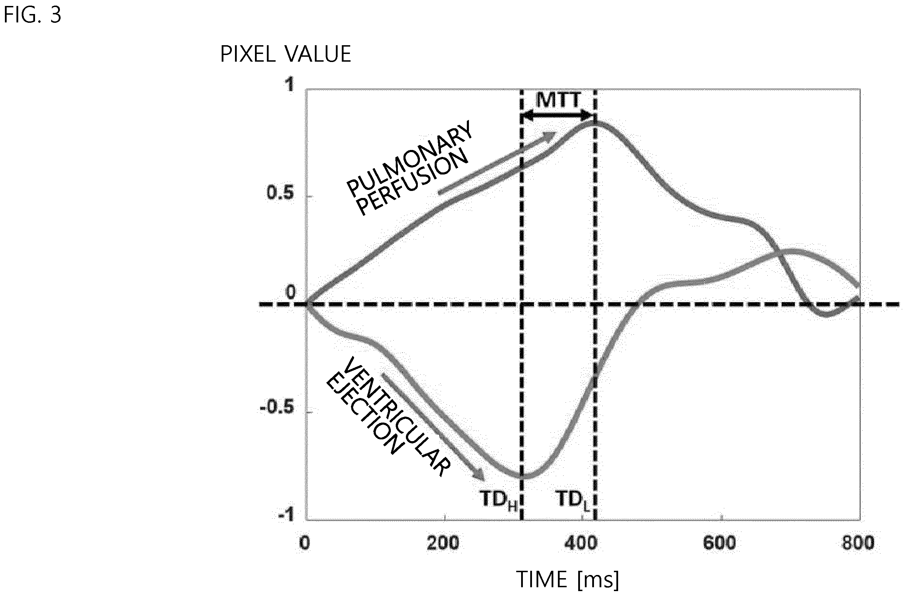

FIG. 3 is a diagram exemplifying a pattern in which values of a first pixel and values of a second pixel change in accordance with discrete points in time for one cardiac cycle of an examinee.

In FIG. 3, the vertical axis shows a pixel value and the horizontal axis shows time, that is, discrete points in time. In FIG. 3, a change of lower pixel values is a change of the values of a first pixel 227 and a change of upper pixel values is a change of the values of a second pixel 217. Although pixel values are shown in a continuous waveform for the convenience of illustration in FIG. 3, these pixel values should be understood as discrete values at discrete points in time. In the embodiment shown in the figure, changes of the values of the first pixel 227 and changes of the values of the second pixel 217 in EIT images obtained from a pig that is an examinee are shown for 800ms that a cardiac cycle of the pig. The pixel values in FIG. 3 are values scaled as values between 0 and 1. The pixel value at 0ms is shown as 0 in FIG. 3 in order to check how much the values of the first pixel 227 and the values of the second pixel 217 change over time in comparison to a reference value under the assumption that the point in time at which an R wave is generated in an ECG waveform (a point in time at which the cardiac cycle starts or a heart starts to contract) is 0ms and the values of the first pixel 227 and the values of the second pixel 217 are all 0, which is the reference value, at the point in time.

According to qualitative analysis of the waveform shown in FIG. 3, a change of the first pixel 227 means a change of the volume of the heart, that is, a change of the amount of blood remaining in the heart, which means that the value of the first pixel 227 increases in the minus direction, the heart contracts and blood flows to lungs through a pulmonary artery from the heart. This process is called ventricular ejection and the point in time at which the value of the first pixel 227 becomes minimum is called a ventricular ejection time TD_{H} or an end systole point in time. After this point in time, the heart no longer contracts and blood increases again in the heart. As shown in the figure, after this point in time, the value of the first pixel 227 approaches back 0 and has a plus value. Meanwhile, a change of the second pixel 217 means a change of the volume of the lungs, that is, a change of the blood remaining in the lungs, which means that as the value of the second pixel (position) 217 increases in the plus direction, blood is ejected from the heart and flows into the lungs. This process is called pulmonary perfusion and the point in time at which the value of the second pixel 217 becomes maximum, that is, the amount of the blood in the lungs becomes maximum as the heart contracts and blood is supplied to the lungs through the pulmonary artery is called pulmonary perfusion time TD_{L}. The value of the second pixel 217 increases as a monotonic function in the embodiment shown in the figures, but, in another embodiment, the value of the second pixel 217 may show a plurality of peak patterns by increasing, decreasing, increasing and decreasing again, and then increasing again and reaching a maximum value. The fact that the value of the second pixel 217 increases and then decreases may be explained by the phenomenon that as the heart contracts, blood goes into the lungs through the pulmonary artery, and in this process, blood flows backward into the heart. The difference between the pulmonary perfusion time TD_{L} and the ventricular ejection time TD_{H} in FIG. 3 is called mean transit time MTT, and this time means time for which blood stops coming out of the heart, but the blood that has come out flows into the lungs.

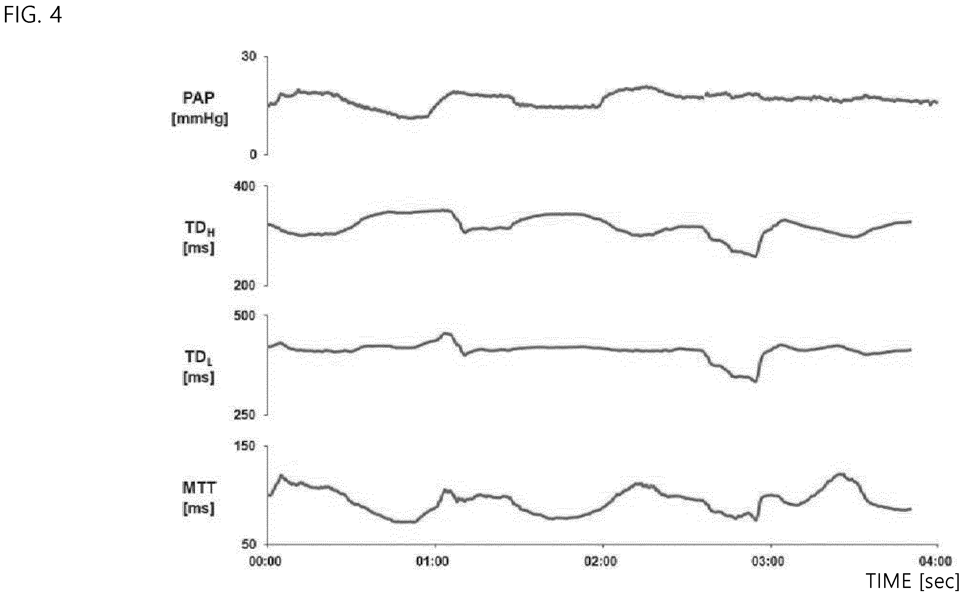

The controller 120 shown in FIG. 1 is described above. The controller 120 may further configured to calculate at least one hemodynamic variable on the basis of a first point in time related to the minimum value of the recognized values of the first pixel 227 and a second point in time related to the maximum value of the recognized values of the second pixel. The minimum value and the maximum value should be understood and analyzed as including not only the minimum value and the maximum value, but all of values in a predetermined difference range from the minimum value and the maximum value. As shown in FIG. 3, the first point in time may be ventricular ejection time TD_{H} and the second point in time may be pulmonary perfusion time TD_{L}. In an embodiment, the controller is further configured to calculate pulmonary artery pressure (pulmonary artery pressure (PAP) on the basis of the first point in time and the second point in time. The controller 120 can calculate pulmonary artery pressure as a function having the first point in time and the second point in time as variables. In an embodiment, the controller 120 can calculate PAP by linearly combining the first point in time and the second point in time as variables. In this embodiment, coefficients for a linear combination of the first point in time and the second point in time may be determined through calibration. When obtaining EIT images, simultaneously, it is possible to measure pulmonary artery pressure through an invasive method and calculate coefficients for a first point in time and a second point in time such that the square of the difference between pulmonary artery pressure expressed as a linear combination and the pulmonary artery pressure measured through the invasive method is minimized by applying least square method. In an embodiment, the controller 120 may be configured to calculate pulmonary artery pressure on the basis of the value obtained by subtracting the first point in time from the second point in time (in this case, the coefficient for the second point in time is 1 and the coefficient for the first point in time is 1), that is, on the basis of mean transit time MTT. Referring to FIG. 4 showing changes over time of ventricular ejection time TD_{H}, pulmonary perfusion time TD_{L}, mean transit time MTT, and pulmonary artery pressure PAP during a plurality of cardiac cycles, it can be seen that the waveform of the mean transit time MTT (MTT: TD_{L} - TD_{H}) closely follows the waveform of the pulmonary artery pressure PAP.

In an embodiment, the controller 120 may be further configured to calculate pulmonary artery pressure on the basis of the first point in time, the second point in time, the minimum value of the values of the first pixel 227, and the maximum value of the values of the second pixel 217. Similar to the embodiment described above, the controller 120 can calculate pulmonary artery pressure on the basis of a first point in time, a second point in time, and a linear combination of a minimum value and a maximum value. In this embodiment, it is also possible to calculate coefficients for the first point in time, the second point in time, the minimum value, and the maximum value such that the square of the difference between pulmonary artery pressure expressed as a linear combination and pulmonary artery pressure measured through an invasive method is minimized by applying least square method. The controller 120 may be further configured to, when the values of the second pixel 217 show two or more peak patterns, calculate pulmonary artery pressure on the basis of the first point in time, the second point in time, the minimum value, the maximum value, peak values of the second pixel 217 at the other peak patterns excluding a peak pattern related to the maximum value of the two or more peak patterns, and points in time related to the peak values. Even in this case, similar to the embodiment described above, the controller 120 can calculate coefficients for the first point in time, the second point in time, the minimum value, the maximum value, the peak values of the second pixel 217, the points in time related to the peak values such that the square of the difference between pulmonary artery pressure expressed as a linear combination and pulmonary artery pressure measured through an invasive method is minimized by applying least square method. The controller 120 may be further configured to, when the values of the first pixel 227 show two or more peak patterns, calculate pulmonary artery pressure on the basis of the first point in time, the second point in time, the minimum value, the maximum value, peak values of the first pixel 227 at the other peak patterns excluding a peak pattern related to the minimum value of the two or more peak patterns, and points in time related to the peak values. Even in this case, similar to the embodiment described above, the controller 120 can calculate coefficients for the first point in time, the second point in time, the minimum value, the maximum value, the peak values of the first pixel 217, the points in time related to the peak values such that the square of the difference between pulmonary artery pressure expressed as a linear combination and pulmonary artery pressure measured through an invasive method is minimized by applying least square method. The controller 120 may be further configured to calculate pulmonary artery pressure on the basis of a first point in time, a second point in time, electrocardiography of an examinee, blood pressure of the examinee, photoplethysmography (PPG) of the examinee, and seismocardiography (SCG) of the examinee. When various data are complexly applied to calculate pulmonary artery pressure, a more accurate pulmonary artery pressure value can be obtained. The controller 120 may be further configured to calculate pulmonary vascular resistance (PVR) using the calculated pulmonary artery pressure. The controller 120 may be further configured to calculate myocardial contractility that is an index showing how much a heart can contract on the basis of the minimum value and the first point in time, that is, the ventricular ejection time TD_{H}. In an embodiment, the controller 120 is configured to calculate myocardial contractility on the basis of a value obtained by dividing the minimum value by the value of the first point in time.

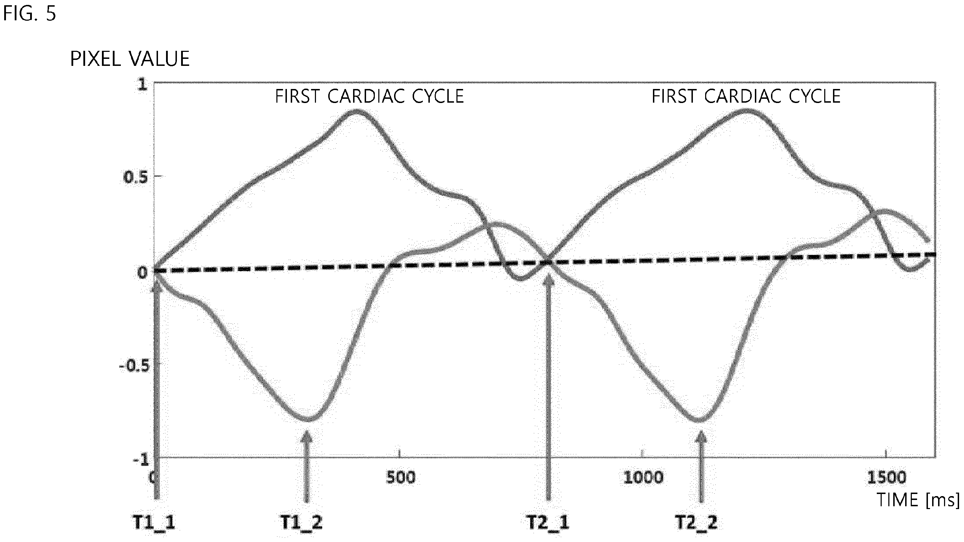

The controller 120 may be further configured to recognize a pixel value corresponding to a first point in time of pixel values in a region corresponding to a heart in EIT images obtained at discrete points in times in a first cardiac cycle of a plurality of cardiac cycles, and a pixel value corresponding a second point in time of pixel values in a region corresponding to the heart of EIT images obtained at discrete points in time in a second cardiac cycle of the plurality of cardiac cycles. In this case, the first point in time is advanced further than the second point in time in terms of time by time corresponding to the cardiac cycle. The controller 120 may be further configured to calculate at least one hemodynamic variable on the basis of a pixel value corresponding to a first point in time and a pixel value corresponding to a second point in time. As shown in FIG. 5 exemplifying a pattern in which values of a first pixel and values of a second pixel change in accordance with discrete points in time during two cardiac cycles, a first point in time and a second point in time are end diastole points in time shown by symbols T1_1 and T2_1 in an embodiment. As known in the art, an end diastole point in time, which is a point in time when a heart is filled with blood the most, is a point in time advanced about 100ms from a point in time, at which an R wave is generated, or a point in time at which a P wave is ended in an ECG waveform. In this embodiment, the controller 120 is further configured to calculate a variation of an end-diastolic ventricular volume (EDW) on the basis of a pixel value corresponding to a first point in time and a pixel value corresponding to a second point in time. In an embodiment, the controller 120 is further configured to calculate myocardial contractility using the calculated variation of an end-diastolic ventricular volume and a variation of a one-time stroke volume (SV) between cardiac cycles. Further, as shown in FIG. 5, in an embodiment, a first point in time and a second point in time are end systole points in time shown by symbols T1_2 and T2_2. As described above, an end systole point in time, which is a point in time corresponding to ventricular ejection time TD_{H}, means time at which blood comes out the most from a heart, that is, ventricles stop contracting. In this embodiment, the controller 120 is further configured to calculate a variation of an end-systolic ventricular volume (ESW) variation on the basis of a pixel value corresponding to a first point in time and a pixel value corresponding to a second point in time. In an embodiment, the controller 120 is further configured to calculate ejection fraction (EF) using the calculated variation of an end-systolic ventricular volume and a variation of one-time stroke volume between cardiac cycles.

The controller 120 may be implemented, in terms of hardware, using at least one of Application Specific Integrated Circuits (ASICs), Digital Signal Processors (DSPs), Digital Signal Processing Devices (DSPDs), Programmable Logic Devices (PLDs), Field-Programmable Gate Arrays (FPGAs), processors, controllers, micro-controllers, and microprocessors. The controller 120 may be implemented as a firmware/software module that can be executed on the hardware platform described above. In this case, the firmware/software module may be implemented by one or more software applications written in an appropriate program language.

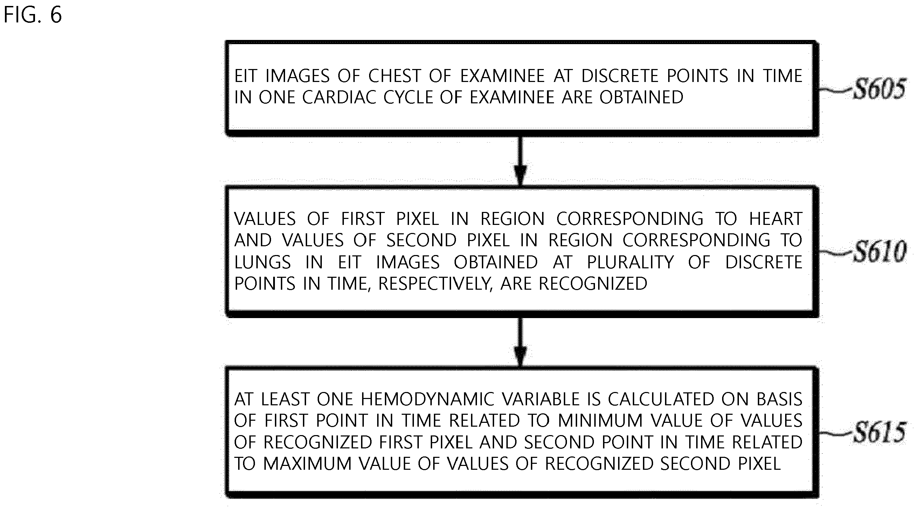

FIG. 6 is a flowchart showing a first embodiment of a method of calculating a hemodynamic variable of an examinee on the basis of ETI.

As shown in FIG. 6, the method starts with a step of obtaining EIT images of the chest of an examinee at discrete points in time in one cardiac cycle of the examinee (S605). In an embodiment, EIT images may be obtained by attaching a plurality of electrodes to the chest of an examinee, obtaining impedance data for the chest of the examinee, and restoring the obtained impedance data. In step S610, values of a first pixel in a region corresponding to a heart and values of a second pixel in a region corresponding to lungs in the EIT images obtained at a plurality of discrete points in time, respectively, are recognized. In this step, it is possible to designate a region 220 showing the heart in the EIT images as a first region of interest 225 and select a first pixel 227 in the first region of interest 225 and it is possible to designate a region 210 showing the lungs in the EIT images as a second region of interest 215 and select a second pixel 217 in the second region of interest 215. The values of a first pixel and the values of a second pixel are all associated with the discrete points in time at which EIT images are obtained. In step S615, at least one hemodynamic variable is calculated on the basis of a first point in time related to the minimum value of the values of the recognized first pixel and a second point in time related to the maximum value of the values of the recognized second pixel. In an embodiment, a first point in time is ventricular ejection time and a second point in time is pulmonary perfusion time. In this step, it is possible to calculate pulmonary artery pressure on the basis of the first point in time and the second point in time. In this step, it is possible to calculate pulmonary artery pressure on the basis of the first point in time, the second point in time, the minimum value, and the maximum value. In this step, when the values of the second pixel show two or more peak patterns, it is possible to calculate pulmonary artery pressure on the basis of the first point in time, the second point in time, the minimum value, the maximum value, peak values of the second pixel at the other peak patterns excluding a peak pattern related to the maximum value of the two or more peak patterns, and points in time related to the peak values. In this step, when the values of the first pixel show two or more peak patterns, it is possible to calculate pulmonary artery pressure on the basis of the first point in time, the second point in time, the minimum value, the maximum value, peak values of the first pixel at the other peak patterns excluding a peak pattern related to the minimum value of the two or more peak patterns, and points in time related to the peak values. In this step, it is possible to calculate pulmonary artery pressure on the basis of the first point in time, the second point in time, electrocardiography of an examinee, blood pressure of the examinee, photoplethysmography of the examinee, and seismocardiography of the examinee. In this step, it is possible to calculate pulmonary vascular resistance using the calculated pulmonary artery pressure. In this step, it is possible to calculate myocardial contractility on the basis of the minimum value and the first point in time.

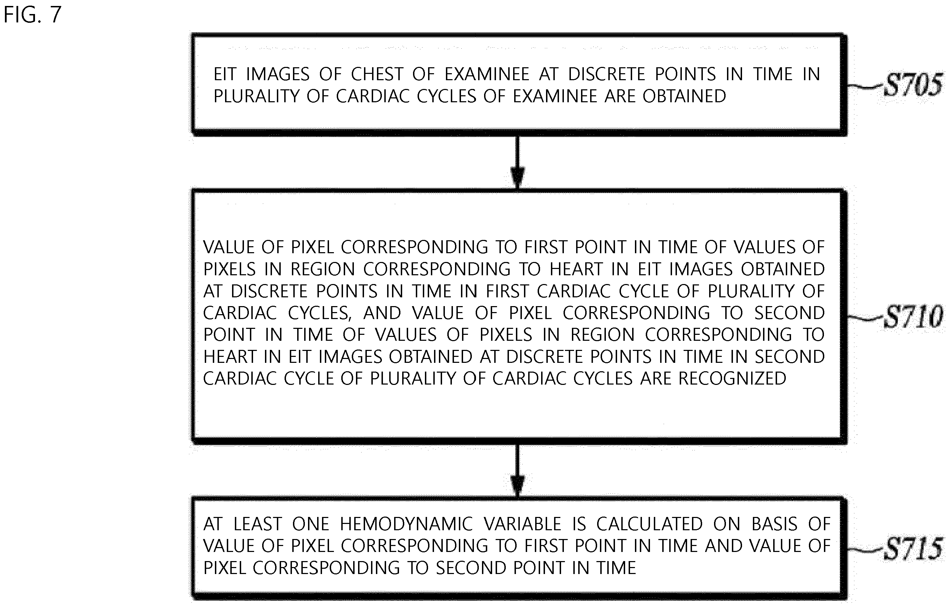

FIG. 7 is a flowchart showing a second embodiment of a method of calculating a hemodynamic variable of an examinee on the basis of ETI.

As shown in FIG. 7, the method starts with a step of obtaining EIT images of the chest of an examinee at discrete points in time in a plurality of cardiac cycles of the examinee (S705). In step S710, a pixel value corresponding to a first point in time of pixel values in a region corresponding to a heart in the EIT images obtained at discrete points in time in a first cardiac cycle of the plurality of cardiac cycles, and a pixel value corresponding to a second point in time of pixel values in a region corresponding to the heart in the EIT images obtained at discrete points in time in a second cardiac cycle of the plurality of cardiac cycles are recognized. In this case, the first point in time is advanced further than the second point in time in terms of time by time corresponding to the cardiac cycle. In step S715, at least one hemodynamic variable is calculated on the basis of a pixel value corresponding to the first point in time and a pixel value corresponding to the second point in time. In an embodiment, the first point in time and the second point in time are end diastole points in time, and in this step of this embodiment, it is possible to calculate a variation of an end-diastolic ventricular volume on the basis of a pixel value corresponding to the first point in time and a pixel value corresponding to the second point in time. In this step, it is possible to calculate myocardial contractility using the calculated variation of an end-diastolic ventricular volume and a variation of a one-time stroke volume between cardiac cycles. In an embodiment, the first point in time and the second point in time are end systole points in time, and in this step of this embodiment, it is possible to calculate a variation of an end-systolic ventricular volume on the basis of a pixel value corresponding to the first point in time and a pixel value corresponding to the second point in time. In this step, it is possible to calculate a stroke rate using the calculated variation of an end-systolic ventricular volume and a variation of a one-time stroke volume between cardiac cycles.

In the above description, when a component is connected or coupled to another component, it should be understood that the component may be not only directly connected or coupled to another component, but may be connected or coupled to another component through one or more other components therebetween. Further, terms for describing the relationship of components (e.g., "between", "among", etc.) should also be analyzed in similar meanings.

In embodiments described herein, arrangement of components shown in the figures may depend on the environment in which the present disclosure is implemented, or requested matters. For example, some components may be omitted or may be combined into one component. The arrangement order or connection of some components may be changed.

Although exemplary embodiments of the present disclosure were illustrated and described above, the present disclosure is not limited to the specific exemplary embodiments, the exemplary embodiments described above may be modified in various ways by those skilled in the art without departing from the scope of the present disclosure described in claims, and the modified examples should not be construed independently from the spirit of the scope of the present disclosure. Accordingly, the technical range of the present disclosure should be determined by only claims.

### [Industrial Applicability]

The present disclosure can non-invasively monitor hemodynamic variables by using electrical impedance tomography.

## Claims

1. A method of calculating a hemodynamic variable of an examinee on the basis of Electrical Impedance Tomography (EIT), the method comprising:
obtaining EIT images of a chest of an examinee at discrete points in time in a cardiac cycle of the examinee; recognizing values of a first pixel in a region corresponding to a heart in the EIT images and values of a second pixel in a region corresponding to lungs - the values of the first pixel are related to the discrete points in time, respectively, and the values of the second pixel are related to the discrete points in time, respectively; and
calculating at least one hemodynamic value on the basis of a first point in time related to a minimum value of the recognized values of the first pixel and a second point in time related to a maximum value of the recognized values of the second pixel.

2. The method of claim 1, wherein the obtaining of EIT images of a chest of an examinee at discrete points in time in a cardiac cycle of the examinee includes: attaching a plurality of electrodes to the chest of the examinee and obtaining impedance data for the chest of the examinee; and restoring the EIT images from the impedance data.

3. The method of claim 1, wherein the recognizing of values of a first pixel in a region corresponding to a heart in the EIT images and values of a second pixel in a region corresponding to lungs - the values of the first pixel are related to the discrete points in time, respectively, and the values of the second pixel are related to the discrete points in time, respectively -, includes designating the region corresponding to the heart in the EIT images as a first region of interest and selecting the first pixel in the first region of interest, and designating the region corresponding to the lungs in the EIT images as a second region of interest and selecting the second pixel in the second region of interest.

4. The method of claim 1, wherein the first point in time is ventricular ejection time and the second point in time is pulmonary perfusion time.

5. The method of claim 1, wherein the calculating of at least one hemodynamic value on the basis of a first point in time related to a minimum value of the recognized values of the first pixel and a second point in time related to a maximum value of the recognized values of the second pixel includes calculating pulmonary artery pressure (PAP) on the basis of the first point in time and the second point in time.

6. The method of claim 5, wherein the calculating of pulmonary artery pressure (PAP) on the basis of the first point in time and the second point in time includes calculating the pulmonary artery pressure on the basis of the first point in time, the second point in time, the minimum value, and the maximum value.

7. The method of claim 5, wherein the values of the second pixel show two or more peak patterns, and
the calculating of pulmonary artery pressure (PAP) on the basis of the first point in time and the second point in time includes calculating the pulmonary artery pressure on the basis of the first point in time, the second point in time, the minimum value, the maximum value, peak values of the second pixel at the other peak patterns excluding a peak pattern related to the maximum value of the two or more peak patterns, and points in time related to the peak values.

8. The method of claim 5, wherein the values of the first pixel show two or more peak patterns, and
the calculating of pulmonary artery pressure (PAP) on the basis of the first point in time and the second point in time includes calculating the pulmonary artery pressure on the basis of the first point in time, the second point in time, the minimum value, the maximum value, peak values of the first pixel at the other peak patterns excluding a peak pattern related to the minimum value of the two or more peak patterns, and points in time related to the peak values.

9. The method of claim 5, wherein the calculating of pulmonary artery pressure (PAP) on the basis of the first point in time and the second point in time includes calculating the pulmonary artery pressure on the basis of the first point in time, the second point in time, electrocardiography (ECG) of the examinee, blood pressure of the examinee, photoplethysmography (PPG) of the examinee, and seismocardiography (SCG) of the examinee.

10. The method of claim 5, wherein the calculating of at least one hemodynamic value on the basis of a first point in time related to a minimum value of the recognized values of the first pixel and a second point in time related to a maximum value of the recognized values of the second pixel further includes calculating pulmonary vascular resistance (PVR) using the calculated pulmonary artery pressure.

11. The method of claim 1, wherein the calculating of at least one hemodynamic value on the basis of a first point in time related to a minimum value of the recognized values of the first pixel and a second point in time related to a maximum value of the recognized values of the second pixel includes calculating myocardial contractility on the basis of the minimum value and the first point in time.

12. A method of calculating a hemodynamic variable of an examinee on the basis of EIT, the method comprising:
obtaining EIT images of a chest of an examinee at discrete points in time in a plurality of cardiac cycles of the examinee;
recognizing a pixel value corresponding to a first point in time of pixel values in a region corresponding to a heart in EIT images obtained at discrete points in times in a first cardiac cycle of a plurality of cardiac cycles, and a pixel value corresponding a second point in time of pixel values in a region corresponding to the heart of EIT images obtained at discrete points in time in a second cardiac cycle of the plurality of cardiac cycles - the first point in time is advanced further than the second point in time in terms of time by time corresponding to the cardiac cycle; and
calculating at least one hemodynamic value on the basis of the pixel value corresponding to the first point in time and the pixel value corresponding to the second point in time.

13. The method of claim 12, wherein the first point in time and the second point in time are end diastole points in time, and
the calculating of at least one hemodynamic value on the basis of the pixel value corresponding to the first point in time and the pixel value corresponding to the second point in time includes calculating a variation of end-diastolic ventricular volume (EDW) on the basis of the pixel value corresponding to the first point in time and the pixel value corresponding to the second point in time.

14. The method of claim 13, wherein the calculating of at least one hemodynamic value on the basis of the pixel value corresponding to the first point in time and the pixel value corresponding to the second point in time further includes calculating myocardial contractility using the calculated variation of end-diastolic ventricular volume and a variation of a one-time stroke volume (SV) between cardiac cycles.

15. The method of claim 12, wherein the first point in time and the second point in time are end systole points in time, and
the calculating of at least one hemodynamic value on the basis of the pixel value corresponding to the first point in time and the pixel value corresponding to the second point in time includes calculating a variation of end-systolic ventricular volume (ESW) on the basis of the pixel value corresponding to the first point in time and the pixel value corresponding to the second point in time.

16. The method of claim 15, wherein the calculating of at least one hemodynamic value on the basis of the pixel value corresponding to the first point in time and the pixel value corresponding to the second point in time further includes calculating ejection fraction (EF) using the calculated variation of end-systolic ventricular volume and a variation of a one-time stroke volume between cardiac cycles.

17. An apparatus for calculating a hemodynamic variable of an examinee on the basis of EIT, the apparatus comprising:
a storage configured to store EIT images - the EIT images are images of a chest of an examinee obtained at discrete points in time in a cardiac cycle of the examinee; and
a controller configured to recognize values of a first pixel in a region corresponding to a heart in the EIT images and values of a second pixel in a region corresponding to lungs - the values of the first pixel are related to the discrete points in time, respectively, and the values of the second pixel are related to the discrete points in time, respectively -, and configured to calculate at least one hemodynamic value on the basis of a first point in time related to a minimum value of the recognized values of the first pixel and a second point in time related to a maximum value of the recognized values of the second pixel.

18. The apparatus of claim 17, wherein the first point in time is ventricular ejection time and the second point in time is pulmonary perfusion time.

19. The apparatus of claim 17, wherein the controller is further configured to calculate pulmonary artery pressure on the basis of the first point in time and the second point in time.

20. The apparatus of claim 19, wherein the controller is further configured to calculate the pulmonary artery pressure on the basis of the first point in time, the second point in time, the minimum value, and the maximum value.

21. The apparatus of claim 17, wherein the controller is further configured to calculate myocardial contractility on the basis of the minimum value and the first point in time.

22. An apparatus for calculating a hemodynamic variable of an examinee on the basis of EIT, the apparatus comprising:
a storage configured to store EIT images - the EIT images are images of a chest of an examinee obtained at discrete points in time in a plurality of cardiac cycles of the examinee; and
a controller configured to recognize a pixel value corresponding to a first point in time of pixel values in a region corresponding to a heart in EIT images obtained at discrete points in times in a first cardiac cycle of a plurality of cardiac cycles, and a pixel value corresponding a second point in time of pixel values in a region corresponding to the heart of EIT images obtained at discrete points in time in a second cardiac cycle of the plurality of cardiac cycles - the first point in time is advanced further than the second point in time in terms of time by time corresponding to the cardiac cycle -, and configured to calculate at least one hemodynamic value on the basis of the pixel value corresponding to the first point in time and the pixel value corresponding to the second point in time.

23. The apparatus of claim 22, wherein the first point in time and the second point in time are end diastole points in time, and
the controller is further configured to calculate a variation of end-diastolic ventricular volume on the basis of the pixel value corresponding to the first point in time and the pixel value corresponding to the second point in time.

24. The apparatus of claim 22, wherein the first point in time and the second point in time are end systole points in time, and
the controller is further configured to calculate a variation of end-systolic ventricular volume on the basis of the pixel value corresponding to the first point in time and the pixel value corresponding to the second point in time.
